# EUROPEAN PATENT SPECIFICATION

(11) **EP 2 281 197 B1**
(45) Date of publication and mention of the grant of the patent: **02.09.2015**
(21) Application number: 09730905.8
(22) Date of filing: 02.04.2009
(51) Int. Cl.: G01N 33/543, G01N 33/58, B82Y 15/00

(54) **METHOD OF DETECTING VERY LOW LEVELS OF ANALYTE WITHIN A THIN FILM FLUID SAMPLE CONTAINED IN A THIN THICKNESS CHAMBER**
VERFAHREN ZUM NACHWEISEN SEHR GERINGER ANALYTNIVEAUS IN EINER DÜNNSCHICHTKAMMER ENTHALTENEN DÜNNFILMFLUIDPROBE
PROCÉDÉ DE DÉTECTION DE TRÈS FAIBLES NIVEAUX D'ANALYTE À L'INTÉRIEUR D'UN ÉCHANTILLON FLUIDE DE FILM MINCE CONTENU DANS UNE CHAMBRE DE PETITE ÉPAISSEUR

(30) Priority: 09.04.2008 US 43571
(43) Date of publication of application: 09.02.2011
(73) Proprietor: Abbott Point Of Care, Inc., Princeton, NJ 08540 (US)
(72) Inventor: WARDLAW, Stephen, C., Lyme CT 06371 (US); LEVINE, Robert, A., Guilford CT 06437 (US)
(74) Representative: Leckey, David Herbert
(86) International application number: PCT/US2009/039283
(87) International publication number: WO 2009/126505

(56) References cited:
- WO-A-00/49415
- WO-A-95/17675
- WO-A-2005/012505
- US-A1- 2007 087 442
- US-B2- 6 866 823
- SUN B ET AL: "Microminiaturized immunoassays using quantum dots as fluorescent label by laser confocal scanning fluorescence detection" JOURNAL OF IMMUNOLOGICAL METHODS, ELSEVIER SCIENCE PUBLISHERS B.V.,AMSTERDAM, NL, vol. 249, no. 1-2, 1 March 2001 (2001-03-01), pages 85-89, XP004317473 ISSN: 0022-1759
- GOLDMAN E R ET AL: "MULTIPLEXED TOXIN ANALYSIS USING FOUR COLORS OF QUANTUM DOT FLUOROREAGENTS" ANALYTICAL CHEMISTRY, AMERICAN CHEMICAL SOCIETY. COLUMBUS, US, vol. 76, no. 3, 1 February 2004 (2004-02-01), pages 684-688, XP001047319 ISSN: 0003-2700

## Description

### BACKGROUND OF THE INVENTION

### 1. Technical Field

This invention relates to a method and apparatus for the detection and quantification of very low levels of a target analyte using an imaging system such as that disclosed in U.S. Patent No. 6,929,953. WO 95/17675 describes a method of assay. WO2005/012505 describes nanoparticle and microparticle based detection of cellular products. In the case of some analytes such as certain hormones, for example TSH, their levels may be as low as several tens of thousands of molecules per micro liter. These extremely low levels can be measured by using the present invention to count the individual molecules of analyte. The invention also has the advantage of being a primary quantitative method, and therefore does not need standardization.

### SUMMARY OF THE INVENTION

In accordance with a first aspect of the invention, there is provided a method as claimed in claim 1.

The method is for the detection and quantification of a defined target analyte disposed, for example, as a thin film biological fluid sample contained in a thin thickness planar chamber typically from about two microns (2µ) to ten microns (10µ) in thickness. The target analyte has at least two epitopes. The method works by binding single molecules of the defined target analyte to an immobile substrate although binders directed against more than one epitope may be employed in an assay. The substrate has a capture antibody or ligand bound to it. The antibodies or ligands are directed against a first epitope or epitopes of the target analyte, and are operable to immobilize the analyte and prevent its diffusion; i.e., to bind the target analyte to the substrate. The bound target analyte is then detected by use of a labeled probe. The probe contains one or more antibodies or ligands bound to its surface, which antibody or ligand is directed against a second epitope or epitopes of the target analyte.

The first and second type epitopes must be spatially located on the target analytes so that the binding of one epitope does not prevent the binding of the second epitope. The term "antibody" and "ligand" shall refer to any substance capable of binding strongly and specifically to a target epitope and shall include immune globulins, aptimers, and any biological binding agents of similar high binding affinity.

This method is suitable for detecting and identifying any target analyte which has at least two accessible epitopes. An example of such a target analyte is TSH (Thyroid Stimulating Hormone). A biological fluid specimen sample, preferably blood plasma or serum, is introduced into a chamber whose surface area dimensions are chosen to permit the maximal countable number of molecules of the target analyte per unit area of the sample as described below.

The bottom or top surface of the chamber is formed from a plastic sheet to which anti-alpha-TSH antibodies are bound, in an amount in excess of that needed to capture the highest amount of the target analyte that is desired to be measured. The capture antibodies must be bound irrevocably to the immobile substrate so that during the assay, the antibodies do not leave the surface to which they are bound. This area is called the capture area.

The blood plasma or serum sample is added to the chamber, and all of the TSH molecules in the sample will bind to the immobile substrate containing the capture antibodies, thereby immobilizing all of the molecules present in the sample. The thin (typically less then ten microns (10µm) chamber thickness allows rapid vertical molecular diffusion so that the diffusion between the two layers of the thin chamber occurs rapidly, allowing all the molecules of the analyte to contact the capture antibody surface. Ideally, the plasma, or other biological fluid being examined, should be clear and free of particles such as cells that might interfere with the binding of analyte or the detection of signal in the assay.

Simultaneously, or after a short initial incubation period, fluorescent nanoparticles which are bound to antibodies, such as anti-beta-TSH antibody, which are specific to a second epitope of the analyte, are added to the sample, also in quantity in excess of that needed to bind the maximal number of molecules to be counted. The nanoparticles are preferably ten to 100 nanometers (10 to 100 nm) in diameter consisting of a Europium fluorescent material, or any detectable nanoparticles, such as those called quantum dots or other fluorescent nanoparticles (Sigma Aldrich, St. Louis, MO, U.S.A. is a supplier). These fluorescent nanoparticles must be sufficiently small and of such density that they will remain in colloidal suspension unless their surface bound antibody becomes attached to an immobilized analyte.

A single fluorescent nanoparticle containing an antibody / ligand directed against the second epitope of the TSH analytes will attach to each TSH molecule that is bound to the substrate. Those fluorescent nanoparticles that are not immobilized by virtue of their attachment to the immobilized analyte will continue to be in colloidal suspension and move due to Brownian motion. To distinguish bound nanoparticles from unbound nanoparticles, the test chamber is imaged under appropriate fluorescent illumination, in the focal plane of the bound particles, after incubation for a period of time which is long enough to give a measurable rise in signal due to the immobile light emitting nanoparticles, as compared to the emission of the moving light emitting nanoparticles which will cause background light due to unbound signal generating nanoparticles. This time of exposure may be adaptively determined by the measuring instrument but limited in its upper extent since it is possible that the areas may have no bound nanoparticles. Those nanoparticles which remain in one location because they are fixed to the substrate will put all of their photons into just a few pixels, while those which "dance" around due to Brownian motion will distribute their brightness over a much larger area, thereby making the detection of the immobile particles possible. A surface area of the chamber which is free of capture antibodies can serve as the control area.

Using this technique, the concentration of nanoparticles in the imaged area should be small enough so that they do not completely overlap and diminish the ability of the sensor to distinguish the immobile particles. The number of individual distinguishable immobile fluorescent particles is therefore equal to the number of molecules of the target analyte contained in the volume of the chamber above or below the capture antibodies within the capture area. Since the volume of the fluid above the control area is relatively small compared to the volume above the immobilized capture antibody or ligand, it may be ignored for purposes of calculating the total volume of the chamber or narrow passage, acting as a diffusion barrier separating the control area from the capture area which may be used to obtain an exact chamber volume over the capture area. Alternatively, an actual impermeable barrier may be employed to separate the capture area from the control area. The maximum number of molecules that may be measured in the contained sample is defined by the capture area of the chamber and the pixel magnification. The concentration of the target analyte will be the number of molecules detected divided by the sample contents in the chamber above the capture area. The volume of the chamber is defined by the known height of the chamber and the area of the sample, which may be defined by the number of pixels within the sample area and the area/pixel magnification factor. Therefore, if the chamber height and magnification are known, the amount of sample volume may also be determined by the instrument performing the analysis. It is necessary that the bound molecules be bound a sufficient distance from each other so that coincidence of signal from the captured labeled nanoparticles avoided. For example, if the fluorescence of a signal contained on a nanoparticle can be detected over an area of 3 to 10 pixels, and the desired image separation of the nanoparticles is at least twice that distance, or about 15 pixels apart, with a magnification yielding an image size of 0.5 microns/pixel, a one square cm of sample area would contain enough resolution for the detection of maximum of about one to two million molecules per chamber. The lower limit of the amount of molecules detected in the chamber is in theory, one, limited of course, by counting statistics. It will be appreciated by one skilled in the art that the thinner the chamber, the greater the discrimination between bound from free labeled target analyte ligand, but the smaller the volume of the sample contained in the chamber. The larger the area of the chamber, the greater the dynamic range, but the longer the time needed to obtain the images of the chamber for analysis.

A 2 cm² chamber, 10 microns (10µ) in height, holding 2 micro liters in the capture area, would be able to detect the presence of a few molecules in this volume. This corresponds to a sensitivity of about 10 attomolar concentration in the source of the sample.

In accordance with a second aspect of the invention, there is provided a method as claimed in claim 11. The sensitivity of the apparatus and method can be linearly increased by increasing the volume of the sample by slowly flowing a 10 microliter to 1,000 microliter sample through the thin chamber, thereby capturing most or all of the molecules in that volume. The flow rate would be in the range of about one to several microliters per second. The assay is done as previously, but the analysis chamber is placed between the sample holding reservoir and the addition of the detection nanoparticles would not be done until completion of the flow and the results reported per volume that flowed through the chamber. The increased volume sample could be pushed through the sample, although the use of an absorbent material in the collection chamber could automate the flow. The sample would flow both over the capture and control areas.

It is, therefore, an object of this invention to provide a method for quantifying the amount of single molecule target analytes in blood plasma or serum placed in an analysis chamber.

It is an object of this invention to provide a method of the character described which involves capturing the target analyte molecules on a surface of a planar thin film sample chamber having at least one transparent surface and optically highlighting the captured molecules so that they may be photometrically counted.

### DESCRIPTION OF THE DRAWINGS

This and other objects, features and advantages of the present invention will become more apparent in light of the detailed description thereof, as illustrated in the accompanying drawing.
FIG. 1 is a schematic plan view of a portion of a thin film sample test chamber for use in assaying a plasma or serum sample for a target analyte, in this case TSH.
FIG. 2 is a view similar to FIG. 1, but showing the test chamber after it has been filled with the plasma or serum sample and a plurality of fluorescent analyte presence reporters.
FIG. 3 is a view similar to FIG. 2 but showing an electronic image of the test chamber when the latter is being imaged for the presence of the target analyte.
FIG. 4 is a schematic plan view of an alternative embodiment of a thin film sample test chamber assembly which includes a higher volume sample source area, a compound thin film test chamber area, and a higher volume sample reception area.
FIG. 5 is a schematic plan view similar to FIG. 4, but showing the sample being moved through the thin film test chamber area.
FIG. 6 is a schematic plan view similar to FIG. 5, but showing the imaging of the thin film test chamber area after the sample has been moved there through.

### DETAILED DESCRIPTION OF THE INVENTION

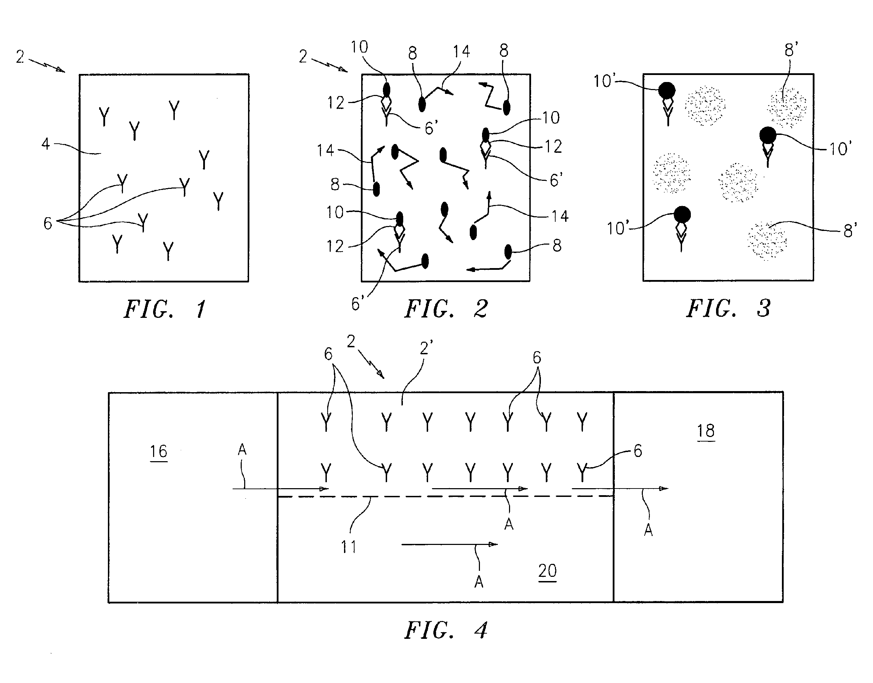

Referring now to FIG. 1 there is shown a portion of a thin film test sampling chamber which is denoted generally by the numeral 2. The test sample being assayed in this case is blood plasma or serum and it is being assayed for the presence of TSH (Thyroid Specific Hormone). The chamber 2 has a surface or wall 4 to which a plurality of ligands 6 is affixed. In this case the ligands 6 will be specific to a first surface epitope of the TSH molecules being assayed.

FIG. 2 shows the chamber 2 after it has been filled with a mixture of the plasma being assayed and fluorescent reporter particles 8. The particles 8 include ligands that are specific to a second epitope on the target analyte so that some of the particles will bond with target analyte molecules prior to being placed in the testing chamber 2. Fluorescent reporter particles that bond to the target analyte molecules 12 are designated by the numeral 10. The free unbound fluorescent reporter particles are designated by the numeral 8 in FIG. 2. The target analytes, in this case TSH, are designated by the numeral 12 in FIG. 2. FIG. 2 shows several of the captured analytes 12 and a number of the free unbound fluorescent reporter particles 8. The unbound particles 8 tend to move in the sample 4 as indicated schematically by arrows 14. This being the case, when the sampling chamber 2 is imaged as shown schematically in FIG. 3, the fluorescent signal from the captured reporter particles (on the target analytes) will be relatively bright in the sample, as indicated by the numeral 10' in FIG. 3, and the fluorescent signal from the free reporter particles will be relatively dim or blurry, as indicated by the numeral 8' in FIG. 3.

Thus the number of captured target analytes in the sample 4 can be easily determined by imaging the sample 4. Since the volume of the sampling chamber 2 is controlled, the volume of the sample 4 in the chamber 2 is known and the target analyte count can be measured in target analyte/sample volume units.

Referring now to FIGS. 4-6, there is shown an embodiment of the device of this invention which is able to sample a larger volume of the sample being assayed. This embodiment includes a sample reservoir 16 in which a larger sample of the plasma or serum to be assayed is placed. The reservoir 16 can hold up to 1 ml, for example, of the sample. The reservoir 16 can have a flexible upper surface which can be depressed so as to compress the sample and pump it through the sample testing chamber component 2 of the assembly. The testing chamber 2 includes a control area 20 which is devoid of capture ligands 6 and the sampling area 2'. This control area is not shown to scale and is much smaller than the capture area or if desired may be connected with a diffusion barrier from the capture area, which includes the analyte capture ligands 6. When the reservoir 16 is compressed, the sample will move in the direction of the arrows A through the sampling area 2' and the control area 20 at the same time. After passing through the areas 2' and 20, the sample will be deposited in a reception reservoir 18 which may contain a sample absorbent, if so desired.

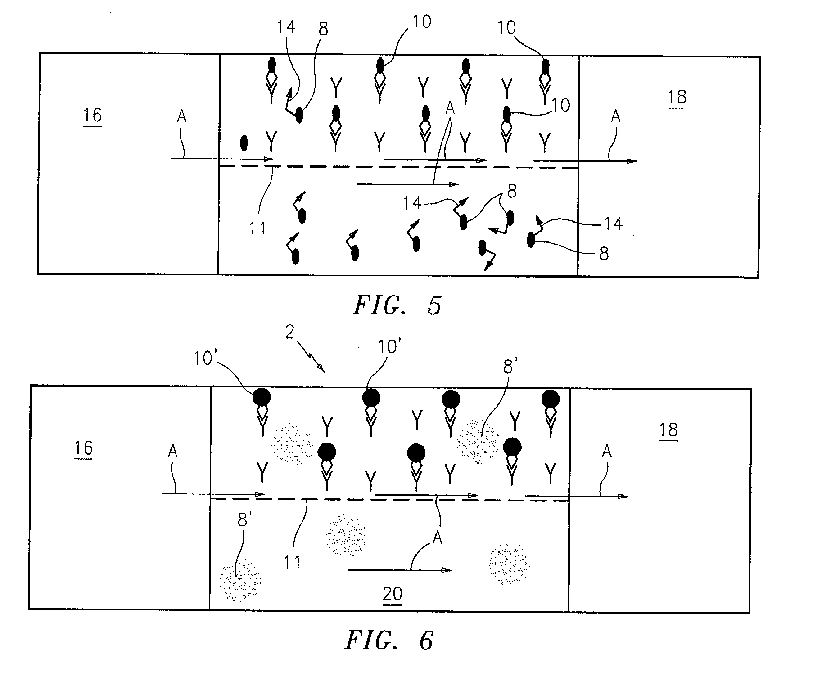

Fig. 6 illustrates the image that will be detected in the sample chamber 2' after the sample has been moved there through. The image will show the bright images 10 of the captured reporter particles, and will show the dimmer and blurrier fluorescent signals 8 from the free or non-captured reporter particles. If the sample test is proven to be valid, then the control area 20 will only include the blurry fluorescent signals 8. The inclusion of the reservoirs 16 and 18 will allow a greater amount of the sample to be assayed, and therefore can provide more valid test results. The broken line 11 in FIGS. 4-6 indicates an impermeable barrier between the sampling area 2' and the control area 20 which prevents sample crossover between the two areas.

Many modifications of this invention with respect to its construction are possible within the description of the invention. They include the area of the assay chamber ranging from 1 mm² to 400 mm², with a height of 2 microns to 10 microns. The localized bound antibodies are preferably placed in a homogeneous pattern, with the adjacent control area having antibodies with no affinity for the desired analyte, or no antibodies at all. It is the control area that is desirable to assure the absence of, or to control for nonspecific detection of, points of higher intensity that do not correspond to a labeled analyte. It is preferable to limit the diffusion of the sample from the control area to the capture area in order to obtain a more accurate volume determination of the amount of sample that is exposed to the capture antibody. It is also possible, if desired to perform as standard curve where multiple concentrations of known analyte are placed in the analysis chamber and analyzed under similar conditions. The number of detectable discrete signals per area imaged in the capture area minus the detectable discrete signals per area imaged in the control area are plotted against the known concentrations of analyte to obtain the standard curve. The results may be used to calculate the concentration of analyte in unknown samples that are analyzed under identical conditions as the standard curve.

Probe signal amplification such as RCAT (rolling circle amplification technology) could be used in place of the nanoparticles since they have the effect of producing localized fluorescent particles.

Since many changes and variations of the disclosed embodiment of the invention may be made without departing from the inventive concept, it is not intended to limit the invention except as required by the appended claims.

## Claims

1. A method for performing an immunoassay of a biological fluid sample for the quantization of a target analyte in a thin film sample chamber wherein the chamber has a thickness of 10µm or less, said method comprising the steps of:
providing a plurality of target analyte specific capture antibodies or ligands (6), sufficient to bind all of the added target analyte (12), which are fixed to a surface (4) of a thin film sample assay chamber (2) or to immobilized structures in the analysis chamber (2), said capture antibodies or ligands (6) being specific to a first epitope or epitopes on target analyte molecules (12) which are present in said biological fluid sample;
filling said sample assay chamber (2) with a mixture of said biological fluid sample and fluorescent nanoparticles (8, 8', 10, 10') coupled to antibodies that selectively bind to a second epitope or epitopes on target analyte molecules (12) which are present in said biological fluid sample; and
imaging said quiescent sample in said sample assay chamber (2) and counting target analyte molecules (12) which are captured by said immobile capture antibodies or ligands (6) and made detectable by imaging the immobilized fluorescent nanoparticles (10, 10') coupled to antibodies that are bound to a second epitope on the immobilized target analyte (12).

2. The method of claim 1 wherein said nanoparticles (8, 8', 10, 10') are Quantum Dots.

3. The method of claim 1 wherein fluorescent nanoparticles (10, 10') which have become immobilized due to binding to captured target analyte molecules in the sample can be photometrically distinguished from free nanoparticles (8, 8') in the sample as a result of movement of the free nanoparticles (8, 8') due to the Brownian motion phenomenon in the sample.

4. The method of claim 1 wherein the fluorescent nanoparticles (10, 10') are Quantum Dots that have become immobilized due to binding to captured target analyte molecules (12) in the sample and can be photometrically distinguished from free nanoparticles (8, 8') in the sample due to movement of the free nanoparticles (8, 8') resulting from the Brownian motion phenomenon in the sample.

5. The method of claim 1 wherein the assayed material is undiluted.

6. The method of claim 1 wherein the chamber (2) contains a control area free of capture antibodies or ligands (6).

7. The method of claim 6 wherein the number of detectable discrete signals per area imaged in the capture area is greater than detectable discrete signals per area imaged in the control area and the difference per area multiplied by the area of the capture area is equal to the number of target analyte molecules captured.

8. The method of claim 6 wherein the number of detectable discrete signals per area imaged in the capture area is greater than detectable discrete signals per area imaged in the control area and is proportional to the number of target analyte molecules captured in the capture area.

9. The method of claim 6 wherein the number of detectable discrete signals per area imaged in the capture area is greater than detectable discrete signals per area imaged in the control area is indicative of the presence of the target analyte in the sample.

10. The method of claim 1 wherein the sample volume applied is greater than the volume of the analysis chamber (2).

11. A method for performing an immunoassay of a biological fluid sample for the quantization of a target analyte in a thin film sample chamber , said method comprising the steps of:
providing a supply of a mixture of said biological fluid sample and fluorescent nanoparticles (8, 8', 10, 10') coupled to antibodies that selectively bind to a second epitope or epitopes on target analyte molecules (12) which are present in said biological fluid sample, said supply have a sample capacity which is greater that the sample capacity of said thin film sample chamber (2);
providing a plurality of target analyte specific capture antibodies or ligands (6), sufficient to bind all of the target analyte (12) in the supply of said mixture, said antibodies or ligands (6) being fixed to a surface (2') of a thin film sample assay chamber (2) or to immobilized structures in the analysis chamber (2), said capture antibodies or ligands (16) being specific to a first epitope or epitopes on target analyte molecules (12) which are present in said biological fluid sample;
moving said mixture from said supply thereof through said sample assay chamber (12) and into a sample reception reservoir (18), whereby said target analyte (12), if present in said sample, will bind to said capture antibodies or ligands (6) in said sample chamber (2); and
imaging said sample assay chamber (2) and counting target analyte molecules (12) which are captured by said immobile capture antibodies or ligands (6) and made detectable by imaging the immobilized fluorescent nanoparticles (10, 10') coupled to antibodies that are bound to said first epitope on immobilized target analyte (12).

12. The method of claim 11 wherein said nanoparticles (8, 8', 10, 10') are Quantum Dots.

13. The method of claim 11 wherein fluorescent nanoparticles (10, 10') which have become immobilized due to binding to captured target analyte molecules (12) in the sample can be photometrically distinguished from free nanoparticles (8, 8') in the sample as a result of movement of the free nanoparticles (8, 8') due to the Brownian motion phenomenon in the sample.

14. The method of claim 11 wherein the fluorescent nanoparticles (10, 10') are Quantum Dots that have become immobilized due to binding to captured target analyte molecules (12) in the sample and can be photometrically distinguished from free nanoparticles (8, 8') in the sample due to movement of the free nanoparticles (8, 8') resulting from the Brownian motion phenomenon in the sample.

15. The method of claim 1 wherein the number of detectable discrete signals per area imaged in the capture area is greater than detectable discrete signals per area imaged in the control area (20) compared to a standard curve performed to calibrate the assay chamber in order to determine the concentration of analyte in the sample.

## Patentansprüche

1. Verfahren zum Durchführen eines Immunassays einer biologischen fluiden Probe zur Quantifizierung eines Zielanalyten in einer Dünnfilmprobenkammer, wobei die Kammer eine Dicke von 10 µm oder weniger aufweist, wobei das Verfahren die Schritte umfasst:
Bereitstellen einer Vielzahl von Fängerantikörpern und - liganden (6), die für den Zielanalyten spezifisch sind, in ausreichender Menge, um den gesamten hinzugefügten Zielanalyten (12) zu binden, die an einer Oberfläche (4) einer Dünnfilmassaykammer (2) oder an immobilisierte Strukturen in der Analysekammer (2) fixiert sind, wobei die Fängerantikörper oder - liganden (6) für ein erstes Epitop oder erste Epitope auf den Zielanalytmolekülen (12) spezifisch sind, welche in der biologischen fluiden Probe vorliegen;
Befüllen der Probenassaykammer (2) mit einer Mischung der biologischen fluiden Probe und fluoreszierenden Nanopartikeln (8, 8', 10, 10'), die an Antikörper gekoppelt sind, die selektiv an ein zweites Epitop oder zweite Epitope auf Zielanalytmolekülen (12) binden, die in der biologischen fluiden Probe vorliegen; und
Abbilden der ruhenden Probe in der Probenassaykammer (2) und Zählen von Zielanalytmolekülen (12), die von den immobilen Fängerantikörpern oder -liganden (6) eingefangen werden und nachweisbar gemacht werden durch Abbilden der immobilisierten fluoreszierenden Nanopartikel (10, 10'), die an Antikörper gekoppelt sind, die an ein zweites Epitop auf dem immobilisierten Zielanalyten (12) gebunden sind.

2. Verfahren nach Anspruch 1, wobei die Nanopartikel (8, 8', 10, 10') Quantenpunkte sind.

3. Verfahren nach Anspruch 1, wobei fluoreszierende Nanopartikel (10, 10'), die aufgrund eines Bindens an eingefangene Zielanalytmoleküle in der Probe immobilisiert wurden, photometrisch von freien Nanopartikeln (8, 8') in der Probe als Ergebnis einer Bewegung der freien Nanopartikel (8, 8') aufgrund des Phänomens der Brownschen Molekularbewegung in der Probe unterschieden werden können.

4. Verfahren nach Anspruch 1, wobei die fluoreszierenden Nanopartikel (10, 10') Quantenpunkte sind, die aufgrund eines Bindens an eingefangene Zielanalytmoleküle (12) in der Probe immobilisiert wurden, und photometrisch von freien Nanopartikeln (8, 8') in der Probe aufgrund einer Bewegung der freien Nanopartikel (8, 8') als Ergebnis des Phänomens der Brownschen Molekularbewegung in der Probe unterschieden werden können.

5. Verfahren nach Anspruch 1, wobei das untersuchte Material unverdünnt ist.

6. Verfahren nach Anspruch 1, wobei die Kammer (2) eine Kontrollfläche frei von Fängerantikörpern oder -liganden (6) enthält.

7. Verfahren nach Anspruch 6, wobei die Anzahl von nachweisbaren diskreten Signale pro Fläche, die in der Einfangfläche abgebildet sind, größer ist als die nachweisbaren diskreten Signale pro Fläche, die in der Kontrollfläche abgebildet sind, und die Differenz pro Fläche multipliziert mit der Fläche der Einfangfläche gleich der Anzahl der eingefangen Zielanalytmoleküle ist.

8. Verfahren nach Anspruch 6, wobei die Anzahl von nachweisbaren diskreten Signale pro Fläche, die in der Einfangfläche abgebildet sind, größer ist als die nachweisbaren diskreten Signale pro Fläche, die in der Kontrollfläche abgebildet sind, und proportional zur Anzahl der in der Einfangfläche eingefangenen Zielanalytmoleküle ist.

9. Verfahren nach Anspruch 6, wobei die Anzahl von nachweisbaren diskreten Signale pro Fläche, die in der Einfangfläche abgebildet sind, größer ist als die nachweisbaren diskreten Signale pro Fläche, die in der Kontrollfläche abgebildet sind, und indikativ für die Anwesenheit des Zielanalyten in der Probe ist.

10. Verfahren nach Anspruch 1, wobei das verwendete Probenvolumen größer ist als das Volumen der Analysekammer (2).

11. Verfahren zum Durchführen eines Immunassays einer biologischen fluiden Probe zur Quantifizierung eines Zielanalyten in einer Dünnfilmprobenkammer, wobei die Kammer eine Dicke von 10 µm oder weniger aufweist, wobei das Verfahren die Schritte umfasst:
Bereitstellen eines Vorrats einer Mischung der biologischen fluiden Probe und fluoreszierenden Nanopartikeln (8, 8' 10, 10'), die an Antikörper gekoppelt sind, die selektiv an ein zweites Epitop oder zweite Epitope auf Zielanalytmolekülen (12) binden, die in der biologischen fluiden Probe vorliegen, wobei der Vorrat eine Probenkapazität aufweist, die größer ist als die Probenkapazität der Dünnfilmprobenkammer (2);
Bereitstellen einer Vielzahl von Fängerantikörpern und - liganden (6), die für den Zielanalyten spezifisch sind, in ausreichender Menge, um den gesamten Zielanalyten (12) in dem Vorrat der Mischung zu binden, wobei die Antikörper oder Liganden (6) an eine Oberfläche (2') einer Dünnfilmassaykammer (2) oder an immobilisierte Strukturen in der Analysekammer (2) gebunden sind, wobei die Fängerantikörper oder -liganden (16) für ein erstes Epitop oder erste Epitope auf den Zielanalytmolekülen (12) spezifisch sind, welche in der biologischen fluiden Probe vorliegen;
Bewegen der Mischung weg von dem Vorrat durch die Probenassaykammer (12) und in ein Probenaufnahmenreservoir (18), wobei der Zielanalyt (12), falls in der Probe vorliegend, an die Fängerantikörper oder -liganden (6) in der Probenkammer (2) bindet; und
Abbilden der Probenassaykammer (2) und Zählen von Zielanalytmolekülen (12), die von den immobilen Fängerantikörpern oder -liganden (6) eingefangen werden und nachweisbar gemacht werden durch Abbilden der immobilisierten fluoreszierenden Nanopartikeln (10, 10'), die an Antikörper gekoppelt sind, die an das erste Epitop auf dem immobilisierten Zielanalyten (12) gebunden sind.

12. Verfahren nach Anspruch 11, wobei die Nanopartikel (8 , 8', 10, 10') Quantenpunkte sind.

13. Verfahren nach Anspruch 11, wobei fluoreszierende Nanopartikel (10, 10'), die aufgrund eines Bindens an eingefangene Zielanalytmoleküle (12) in der Probe immobilisiert wurden, photometrisch von freien Nanopartikeln (8, 8') in der Probe als Ergebnis einer Bewegung der freien Nanopartikel (8, 8') aufgrund des Phänomens der Brownschen Molekularbewegung in der Probe unterschieden werden können.

14. Verfahren nach Anspruch 11, wobei die fluoreszierenden Nanopartikel (10, 10') Quantenpunkte sind, die aufgrund eines Bindens an eingefangene Zielanalytmoleküle (12) in der Probe immobilisiert wurden, und photometrisch von freien Nanopartikeln (8, 8') in der Probe aufgrund einer Bewegung der freien Nanopartikel (8, 8') als Ergebnis des Phänomens der Brownschen Molekularbewegung in der Probe unterschieden werden können.

15. Verfahren nach Anspruch 1, wobei die Anzahl der nachweisbaren diskreten Signale pro Fläche, die in der Einfangfläche abgebildet sind, größer ist als die nachweisbaren diskreten Signale pro Fläche, die in der Kontrollfläche (20) abgebildet sind, verglichen mit einer Standardkurve, welche erstellt wurde, um die Assaykammer zu kalibrieren, um die Konzentration des Analyten in der Probe zu bestimmen.

## Revendications

1. Procédé de réalisation d'un immunodosage d'un échantillon de fluide biologique pour la quantification d'un analyte cible dans une chambre d'échantillon de film mince, dans lequel la chambre a une épaisseur de 10 µm ou moins, ledit procédé comprenant les étapes consistant à :
fournir une pluralité d'anticorps ou ligands de capture (6) spécifiques à l'analyte cible à suffisance pour se lier à la totalité de l'analyte cible ajouté (12), qui sont fixés à une surface (4) d'une chambre de dosage d'échantillon de film mince (2) ou à des structures immobilisées dans la chambre d'analyse (2), lesdits anticorps ou ligands de capture (6) étant spécifiques à un ou des premiers épitopes sur les molécules de l'analyte cible (12) qui sont présentes dans ledit échantillon de fluide biologique ;
remplir ladite chambre de dosage d'échantillon (2) par un mélange dudit échantillon de fluide biologique et de nanoparticules fluorescentes (8, 8', 10, 10') couplées à des anticorps qui se lient sélectivement à un ou des seconds épitopes sur les molécules d'analyte cible (12) qui sont présentes dans ledit échantillon de fluide biologique ; et
imager ledit échantillon au repos dans ladite chambre de dosage d'échantillon (2) et compter les molécules d'analyte cible (12) qui sont capturées par lesdits anticorps ou ligands de capture immobiles (6) et rendues détectables par imagerie des nanoparticules fluorescentes immobilisées (10, 10') couplées à des anticorps qui sont liés à un second épitope sur l'analyte cible immobilisé (12).

2. Procédé selon la revendication 1, dans lequel lesdites nanoparticules (8, 8', 10, 10') sont des points quantiques.

3. Procédé selon la revendication 1, dans lequel des nanoparticules fluorescentes (10, 10') qui se sont immobilisées en raison de la liaison à des molécules capturées d'analyte cible de l'échantillon peuvent être distinguées par voie photométrique des nanoparticules libres (8, 8') de l'échantillon à la suite du mouvement des nanoparticules libres (8, 8') dû au phénomène de mouvement brownien dans l'échantillon.

4. Procédé selon la revendication 1, dans lequel les nanoparticules fluorescentes (10, 10') sont des points quantiques qui se sont immobilisés en raison de la liaison à des molécules d'analyte cible capturées (12) de 1"échantillon et peuvent être distinguées par voie photométrique des nanoparticules libres (8, 8') de l'échantillon en raison du mouvement des nanoparticules libres (8, 8') résultant du phénomène de mouvement brownien dans l'échantillon.

5. Procédé selon la revendication 1, dans lequel le matériau dosé n'est pas dilué.

6. Procédé selon la revendication 1, dans lequel la chambre (2) contient une zone témoin exempte d'anticorps ou ligands de capture (6).

7. Procédé selon la revendication 6, dans lequel le nombre de signaux discrets détectables par surface imagée dans la zone de capture est supérieur au nombre de signaux discrets détectables par surface imagée dans la zone témoin et la différence par surface multipliée par la surface de la zone de capture est égale au nombre de molécules d'analyte cible capturées.

8. Procédé selon la revendication 6, dans lequel le nombre de signaux discrets détectables par surface imagée dans la zone de capture est supérieur au nombre de signaux discrets détectables par surface imagée dans la zone témoin et est proportionnel au nombre de molécules d'analyte cible capturées dans la zone de capture.

9. Procédé selon la revendication 6, dans lequel le nombre de signaux discrets détectables par surface imagée dans la zone de capture est supérieur au nombre de signaux discrets détectables par surface imagée dans la zone témoin et est indicatif de la présence de l'analyte cible dans l'échantillon.

10. Procédé selon la revendication 1, dans lequel le volume d'échantillon appliqué est supérieur au volume de la chambre d'analyse (2).

11. Procédé de réalisation d'un immunodosage d'un échantillon de fluide biologique pour la quantification d'un analyte cible dans une chambre d'échantillon de film mince, dans lequel la chambre a une épaisseur de 10 µm ou moins, ledit procédé comprenant les étapes consistant :
fournir une alimentation d'un mélange dudit échantillon de fluide biologique et de nanoparticules fluorescentes (8, 8', 10, 10') couplées à des anticorps qui se lient sélectivement à un ou des seconds épitopes sur les molécules d'analyte cible (12) qui sont présentes dans ledit échantillon de fluide biologique, ladite alimentation ayant une capacité d'échantillon qui est supérieure à la capacité d'échantillon de ladite chambre d'échantillon de film mince (2) ;
fournir une pluralité d'anticorps ou ligands de capture (6) spécifiques à l'analyte cible à suffisance pour se lier à la totalité de l'analyte cible (12) dans l'alimentation dudit mélange, lesdits anticorps ou ligands (6) étant fixés à une surface (2') d'une chambre de dosage d'échantillon de film mince (2) ou à des structures immobilisées dans la chambre d'analyse (2), lesdits anticorps ou ligands de capture (16) étant spécifiques à un ou des premiers épitopes sur les molécules d'analyte cible (12) qui sont présentes dans ledit échantillon de fluide biologique ;
déplacer ledit mélange de sa dite alimentation à travers ladite chambre de dosage d'échantillon (12) et dans un réservoir récepteur d'échantillon (18), moyennant quoi ledit analyte cible (12), s'il est présent dans ledit échantillon, se liera auxdits anticorps ou ligands de capture (6) dans ladite chambre d'échantillon (2) ; et
imager ladite chambre de dosage d'échantillon (2) et compter les molécules d'analyte cible (12) qui sont capturées par lesdits anticorps ou ligands de capture immobiles (6) et rendues détectables par imagerie des nanoparticules fluorescentes immobilisées (10, 10') couplées à des anticorps qui sont liés audit premier épitope sur l'analyte cible immobilisé (12).

12. Procédé selon la revendication 11, dans lequel lesdites nanoparticules (8, 8', 10, 10') sont des points quantiques.

13. Procédé selon la revendication 11, dans lequel des nanoparticules fluorescentes (10, 10') qui se sont immobilisées en raison de la liaison à des molécules capturées d'analyte cible (12) de l'échantillon peuvent être distinguées par voie photométrique de nanoparticules libres (8, 8') de l'échantillon à la suite du mouvement des nanoparticules libres (8, 8') dû au phénomène de mouvement brownien dans l'échantillon.

14. Procédé selon la revendication 11, dans lequel les nanoparticules fluorescentes (10, 10') sont des points quantiques qui se sont immobilisés en raison de la liaison à des molécules d'analyte cible capturées (12) de l'échantillon et peuvent être distinguées par voie photométrique de nanoparticules libres (8, 8') de l'échantillon en raison du mouvement des nanoparticules libres (8, 8') résultant du phénomène de mouvement brownien dans l'échantillon.

15. Procédé selon la revendication 1, dans lequel le nombre de signaux discrets détectables par surface imagée dans la zone de capture est supérieur au nombre de signaux discrets détectables par surface imagée dans la zone témoin (20) en comparaison d'une courbe standard effectuée pour étalonner la chambre de dosage afin de déterminer la concentration d'analyte dans l'échantillon.
